# EUROPEAN PATENT SPECIFICATION

(11) **EP 3 712 826 B1**
(45) Date of publication and mention of the grant of the patent: **14.05.2025**
(21) Application number: 18875620.9
(22) Date of filing: 13.11.2018
(51) Int. Cl.: G06Q 10/06

(54) **SYSTEM AND METHOD FOR PERFORMING AND/OR DOCUMENTING AN ASSISTED PROFESSIONAL PROCEDURE**
SYSTEM UND VERFAHREN ZUR DURCHFÜHRUNG UND/ODER DOKUMENTATION EINES UNTERSTÜTZTEN PROFESSIONELLEN PROZESSES
SYSTÈME ET PROCÉDÉ POUR LA RÉALISATION ET/OU LA DOCUMENTATION D'UNE OPÉRATION PROFESSIONNELLE ASSISTÉE

(30) Priority: 13.11.2017 BR 102017024387
(43) Date of publication of application: 23.09.2020
(73) Proprietor: ILTEC - LUBECK TECNOLOGIA LTDA., 13301-510 Sao Paulo (BR)
(72) Inventor: PINHO, Mauro de Souza Leite, 89203-230 Joinville (BR); BRAMANTE, Thiago Magalhaes, 18119-372 Votorantim (BR); PEDROSO, Miguel Angelo, 13305-430 Itu (BR)
(74) Representative: Grünecker Patent- und Rechtsanwälte PartG mbB
(86) International application number: PCT/BR2018/050418
(87) International publication number: WO 2019/090410

(56) References cited:
- WO-A1-2016/119034
- WO-A1-2016/119034
- US-A1- 2003 083 563
- US-A1- 2012 182 244
- US-A1- 2012 182 244
- US-A1- 2018 336 500

## Description

### Field of the Invention

The present invention describes a system and process for real-time performing and documenting remotely assisted professional procedure. Specifically, the present invention discloses a system comprising: at least one signal management device comprising at least one of capture means, recording means, reproducing means, command means and/or signal transmission means; and at least one signal management server comprising at least one of a storage means and/or a communication addressing means. The present invention is in the fields of medicine, distance education, specialized consulting and electrical engineering.

### Background of the Invention

Communication devices are present in the day by day of the population, such as computers, PDAs, tablets, smartphones etc. With this advance, it became possible to interconnect several users who can exchange text messages, audio messages, recorded and real-time video messages, among other variations. These advances enable users to communicate from various places on the planet.

Such devices are also applied on an industrial scale wherein they connect several types of equipment, such as industrial process machines etc., communicating them with the appropriate control systems, so that said actions can be performed remotely, i.e. with no need for an on-site technician.

Other applications include the field of education, in which classes are offered remotely to students on a non-face-to-face basis in environments and communicate with professionals/teachers in the chosen field. There are also cases wherein said devices are used in medical areas, such as in training new professionals. In these applications, a specialized physician remotely assists a physician who is using an equipment, or even performing surgery. In these situations, communication devices must be extremely reliable, and also provide the connection with more than one expert remotely accompanying the process, so as to further improve the assistance to the physician performing said task locally - increasing the reliability of the procedure itself even in places having either difficulty of access or absence of professionals specialized in the procedure being performed.

In the search for the prior state in scientific and patent literature, some documents dealing with the subject matter were found, being only partially relevant in the context of the present invention:

US2003083563 describes a system for the data acquisition and transmission obtained through local medical devices, wherein unprocessed data is transmitted to a remote base, where it is processed by a skilled/specialized physician and forwarded to local device with appropriate changes. Such a solution, however, implies that the device allocated at the remote base requires high data processing capacity for assembly the sent image. Moreover, such a solution does not allow other users to receive and/or change data sent by the local base, as it is peer-to-peer communication. However, the solution proposed in US2003083563, since it refers to the performance of medical examinations, does not have a high reliability in the transmission of information, which would imply too many consequences in the case of conducting real-time surgeries.

WO2016119034 describes a system, device and process for the performance and documentation of real-time remote assisted professional procedure. The system of said invention comprises: a local device or equipment, where the medical procedure is performed, having means for receiving, reproducing and streaming video and audio signals; one or more remote devices or equipments having means for receiving, reproducing and/or online editing and streaming of video and audio to the device. Such a solution, however, requires large bandwidth for remote assistance, since video data is sent over both channels between the communicating devices. In addition, a connection to a network server is required during communication, delaying response time for assistance.

US2012/182244 A1 discloses a pan/till/zoom command tool for controlling camera movements, allowing the remote user to get a more appropriate image of the local room.

WO2016/119034 A1 refers to a graphical editing tool, where the remote user edits/ modifies a received image, indicating to the surgeon locations where an incision should be made. When editing is finished the edited image is sent to the surgeon.

Equipment and systems for assisting professional/surgical procedures through remote professional/tutorial support, which provide the transmission of image and/or voice data online or almost online, are also known. However, said equipments require sophisticated facilities for data connection and/or transmission, have a complex structure and are often inaccessible to the facilities where the procedure is performed. In addition, said equipments do not provide for online editing of video images by the remote professional/tutor, so as the locally performing professional/physician receives online video information edited together with the audio.

The currently known equipments and systems enable a type of communication requiring high processing capacity on the remote base, i.e. the system must be robust, implying a high cost of implementation.

Therefore, from what is clear from the researched literature, no document is found anticipating or suggesting the teachings of the present invention, so that the solution proposed here, in the eyes of the inventors, has novelty and inventive step over to the prior art.

### Summary of the Invention

Thus, the present invention aims at solving the problems of the prior art by a system and process for performing and documenting real-time remote assisted professional procedure, in which a simplified procedure is provided for the communication between an assistant consultant and a professional user performing the procedure, where a channel for communication with controlled and/or reduced information flow is generated, thereby providing greater real-time reliability during the procedure. The system comprises: at least one signal management device (14) communicating to at least one signal management server (16) which comprises at least one of between a storage means (164) and/or a communication addressing means (162).

In a first aspect, the present invention discloses a system for the performing and/or documenting of real-time remotely assisted professional procedure comprising:
a. at least one signal management device (14) comprising at least one between a capture means (31), a recording means, a reproducing means (22), a command means (36) and/or a signal transmission means (35); and
b. at least one signal management server (16) comprising at least one between a storage means (164) and/or a communication addressing means (162);
wherein,
- the signal management device (14) and the signal management server (16) communicate each other through a communication channel which is able to traffic at least one signal (SU).

In a second aspect, the present invention discloses a process for performing and/or documenting real-time remotely assisted professional procedure comprising at least the following steps:
a. capturing of at least one signal (SE) from at least one signal source (10), by a capture means (31), in a signal management device (14);
b. sending at least one signal (SU) by the signal management device (14) to at least one signal management server (16) configured to receive the signal (SU).

These and other aspects of the invention will be readily appreciated by those skilled in the art and companies having an interest in the field, and will be described in sufficient detail for their reproduction in the following description.

### Brief Description of the Figures

In order to better define and to clarify the contents of the present patent application, the following figures are showed:
Fig. 1 shows an embodiment of the system of the present invention.
Fig. 2 shows an embodiment of the signal management device (14) and its communication with the signal management server (16) and the access device (12).
Fig. 3 shows a block diagram showing the flow of signals in the system of the present invention.
Figs. 4 and 5 show an embodiment of the reproducing means (22).

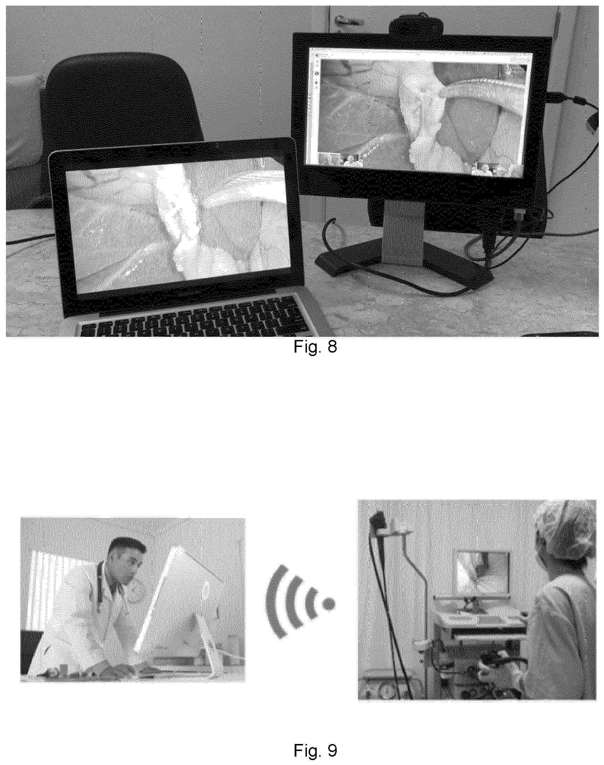

Figs. 6 to 8 show an embodiment of the system functioning, the short delay of information traffic being highlighted.
Fig. 9 shows the scheme of direct and independent connection of the access device (12) with the signal management device (14).

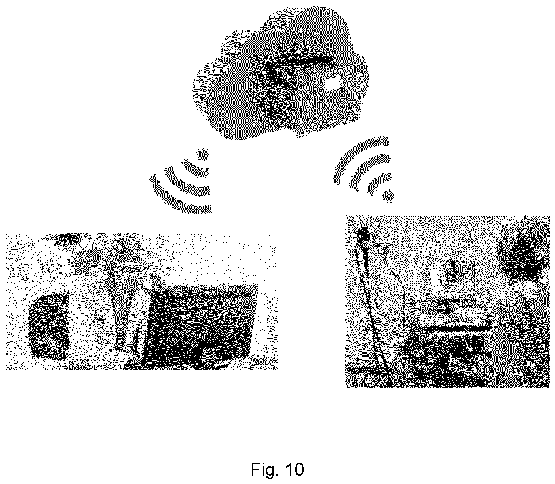

Fig. 10 shows the scheme of signal storage (SS) in a signal management server (16), and the subsequent access of this signal (SS) by someone else.

### Detailed Description of the Invention

The following descriptions are showed by way of example and are not limiting the scope of the invention and will more make the subject matter of the present patent application more clearly understood.

In a first aspect the present invention discloses a system for the performing and/or documenting of real-time remotely assisted professional procedure comprising:
a. at least one signal management device (14) comprising at least one between a capture means (31), a recording means (34), a reproducing means (22), a command means (36) and/or a signal transmission means (35); and
b. at least one signal management server (16) comprising at least one between a storage means (164) and/or a communication addressing means (162);
wherein,
- the signal management device (14) and the signal management server (16) communicate each other through a communication channel which is able to traffic at least one signal (SU).

The signal management device (14) is capable of receiving signals by the capture means (31) so as to be distributed to its other components in order to record, reproduce and/or transmit such signals to other devices, servers or other signal receiving means.

The signal management device (14) communicates with other devices via a connection made by the signal management server (16) for this purpose, said device using a command means (36) and/or transmission means (35) for emitting a signal (SU) towards the signal management server (16). Upon receiving the signal (SU) and another signal (SC) from another device, the server authenticates the connection and creates a traffic channel (CT) between both devices.

Capture means (31) comprises any component or set of components capable of receiving signals (SE) from at least one signal source (10) via signal inputs, for example, a capture card, video capture card, USB card for audio, video and image capture, devices for storage and/or audiovisual file sharing, tuners etc. In one embodiment, the signal management device (14) generates a signal (SS) from the input signal (SE). In one embodiment, the capture means (31) further comprises the generation of at least one signal (SS), where such signal (SS) is directed to the other device to which the signal management device (14) is communicating. In one embodiment, the signal (SS) is a signal generated from the input signal (SE), where the device may: I - replicate the input signal (SE) for generating signal (SS); II - process the input signal (SE) by means of mathematical tools to adapt the signal quality relative to the connection band, and generate the signal (SS); III - apply correction filters, for example, audio and/or video filters on the input signal (SE) thereby generating the signal (SS); IV - combine several input signals (SE) to merge them into one signal (SS); V - combine several input signals (SE) with signals from other sources to join them into one signal (SS); and VI - a combination of these mentioned tasks.

The recording means (34) comprises any element or set of elements capable of recording the signals (SE) in order to store it in the signal management device (14) itself generating a file, regardless of having to send it to other destination or erase it from its memory, for example memories RAM, ROM, DRAM, DIP, SIMM, FPM, EDO, DIMM, SDRAM, RIM, PC100, DDR, DUAL CHANNEL, TRIPLE CHANNEL, XDR DRAM, PROM, EPROM, EEPROM, SSD, FRAM, Flash, quantum storage, volatile memories, nonvolatile memories, optical disks, magnetic tapes, data storage devices, data storage media, dynamic memories, static memories etc.

In one embodiment, the recording means (34) comprises a compilation of at least one signal (SE) and the generation of at least one file for subsequent sending to and storage on the signal management server (16). In this embodiment, the file is sent as a signal (SU).

In one embodiment, the signal management device (14) comprises a component configured for performing data exchange, being able to send and receive data, for example, as sending/receiving command signals for handshake, sending/receiving signals from compiled files, sending/receiving communication signals etc. For exemplification purposes, this component comprises at least one command means (36) and/or a signal transmission means (35), which enable the signal management device (14) to communicate with the signal management server (16) and/or with other devices in order to send the signals (SU), for example, antennas, physical lines, transmitter compounds, modulators, amplifiers, multiplexers, a combination thereof etc.

The signal (SU) as referred to above comprises the ability to be one between a command signal for authentication in signal management server (16), a file comprising the contents of at least one signal (SE), a video, audio, text signal, a command signal for signal selection (SE) or a combination thereof. In one embodiment, the signal (SU) comprises a command signal used for handshake and/or a compiled file containing all or part of the content coming from the input signal (SE).

The reproducing means (22) enables the reproduction of the signals received by the signal management device (14), so as to provide the user of the signal management device (14) with the desired information, for example a monitor, display, video and audio player, electric to audio signal conversion devices, electric to images and video signal conversion devices, sound cards, a combination thereof etc.

The signal management server (16) comprises a storage means (164) for storing sending signals (SU) and/or a communication addressing means (162) with the ability to authenticate communication between two devices. In one embodiment, the communication addressing means (162) creates a traffic channel (CT) outside the signal management server (16). In another embodiment, signal management server (16) comprises a traffic channel (CT) created by communication addressing means (162). In one embodiment, the traffic channel (CT) generated by the server is Peer-to-Peer type.

The storage means (164) enables the management server to store received signals, for example, a database, data centers, databank, local storage servers, an intercommunication between storage servers, a cloud server, a combination thereof etc. In addition, the storage means (164) enables the reproduction of these stored signals when requested.

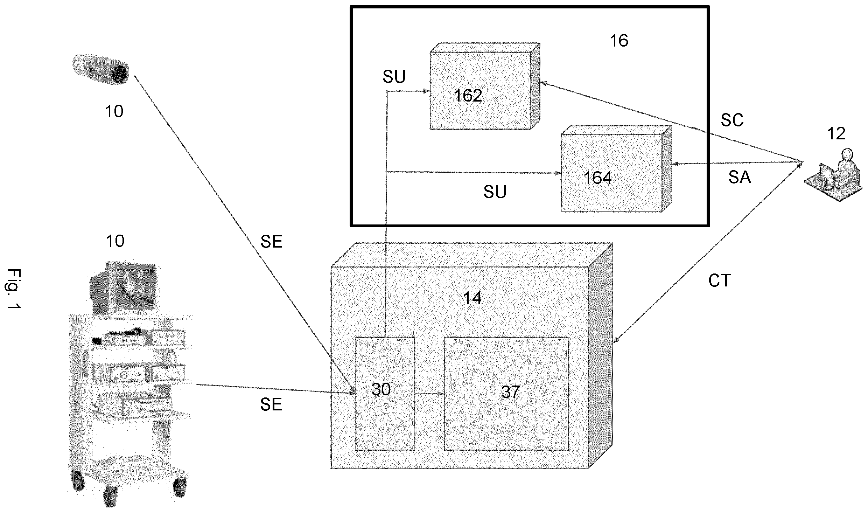

In one embodiment, the system further comprises an access device (12), provided with user and/or consultant, which communicated with the signal management server (16) and is capable of communicating with the signal management device (14). In this embodiment, the access device (12) is capable of sending a signal (SC), by a sending means, for requesting the addressing to the server to which authentication between signal (SC) and signal (SU), coming from the signal management device (14) is verified, as shown in Fig. 1. In one embodiment, the storage means (164) comprises the ability to store signals from the signal management device (14). In another embodiment, the storage means (164) comprises the ability to store signals from the signal management device (14) and the access device (12).

The means for sending at least one signal (SC) is a component or a set of components enabling the communication between the access device (12) and the signal management server (16), and transmits the desired information, such as sending the signals (SU), by, for instance, antennas, physical lines, transmitter compounds, modulators, amplifiers, multiplexers, a combination thereof etc.

In one embodiment, the signal management device (14) compiles at least one signal (SE), generating at least one file for subsequent sending and storing on the signal management server (16), wherein this file can be accessed by the user and/or consultant via the access device (12). For illustration purposes, without limiting the scope of the invention, the signal management device (14) can store a file on the signal management server (16), which is available for access by the user and/or by the consultant.

In one embodiment, the signal management device (14) further comprises a processing means (33) configured to enable the overlapped reproduction of the SS and SA signals, thereby assisting the user performing the professional procedure using the signal management device (14). Sadi processing enables the user performing the professional procedure to explain possible operating paths of the professional procedure, and further enables a second perspective during the professional procedure.

In another embodiment, the processing means (33) is configured to enable reproduction of the sent signals (SS). In another embodiment, the processing means (33) is configured to enable reproduction of the received signals (SA).

The processing means (33) comprises the ability to be any component or set of components capable of manipulating the signals (SS) and signals (SA) for the reproduction of both signals, for example a processor, CPU, GPU, central processor combined with peripheral processors, central processing, processing cores, a combination thereof etc.

Thereby, the communication addressing means (162) performs verification of the communication authenticity between two devices, that is, once authentication is performed, it enables the devices to connect and communicate with no direct restriction of the communication between devices, for example, parity check systems, APIs, gateways, RTC protocols, SIP protocols, a combination thereof etc. For illustration purposes, this communication is Peer-to-Peer type, and protocols adapted for this purpose may be used.

In order to enable this communication between the devices, the server addressing means (162) generates the traffic channel (CT) right after verification of the authentication between the (SC) and (SU) signals. In one embodiment, through the authentication, the signal management server (16) comprises an authentication protocol between the signal management device (14) and the access device (12).

In one embodiment, the traffic channel (CT) comprises communication between the signal management device (14) and the access device (12), so that communication between the devices is not accessed by third parties without authorization, and appropriate authentication. This direct communication between devices results in safer and faster communication between devices, thus the received signal has a higher reliability with the sent signal.

In addition, the signals (SA) originating from the access device (12) are sent in signal sets in order to enable greater stability in the sent signal flow, according to the bandwidth available on the access device (12). In this way, communication occurs in an uninterrupted way at a faster rate than prior art systems.

In one embodiment, the access device (12) comprises the sending of at least one signal (SA) towards the signal management device (14), wherein the signal (SA) comprises at least one of video signal, audio signal, graphic coordinates, overlapping images, command signal for signal selection (SE), command signal for intensity control of audio, text, or a combination thereof.

In one embodiment, the access device (12) comprises viewing all received signals (SS). In one embodiment, the access device (12) provides alternating between displaying the signals (SS). In a further embodiment, the access device (12) sends an electrical command to the signal management device (14) for selecting and changing the signal (SE) desired for transmission. In a further embodiment, the access device (12) sends an electrical command to the signal management server (16) for selecting and changing the signal (SE) desired for transmission.

In one example, the user and/or consultant, via the access device (12), sends the signal (SA) containing graphic coordinates, which have been implemented from the received signal (SS), such as the signal management device (14) performs a processing to overlap said signals, where the graphic coordinate is overlapped on the signal (SS) generated by the signal management device (14) itself.

In one embodiment, the system for performing and/or documenting real-time remotely assisted professional procedure is applied to professional procedures such as surgical procedures, clinical examinations, clinical analysis and/or medical education.

In a second aspect the present invention discloses a process for performing and/or documenting real-time remotely assisted professional procedure comprised of at least the following steps:
a. capturing of at least one signal (SE) from at least one signal source (10), by a capture means (31), in a signal management device (14);
b. sending at least one signal (SU) by the signal management device (14) to at least one signal management server (16) configured to receive the signal (SU).

The step of capturing at least one signal (SE) enables the reception of these signals in the signal management device (14), so as they are available for signal handling, storage and sending of the signals.

The step of sending at least one signal (SU) by the signal management device (14) to at least one signal management server (16) enables interaction between the signal management device (14) and the signal management server (16), so as the signal (SU) is distributed within the signal management server (16) according to its function. In one embodiment, the signal (SU) is an electrical command, wherein within the signal management server (16) an authentication with another signal (SC) from an access device (12) is required. In one embodiment, the signal (SU) is a file or parts of files for storage, comprising the content received by the signal (SE).

The process further comprises a step of sending a signal (SC) by an access device (12) followed by the generation of a traffic channel (CT) by the signal management server (16), connecting the access device (12) to the signal management device (14). Traffic channel (CT) generation occurs right after the authentication verification between the (SC) and (SU) signals. This traffic channel (CT) generates communication between the signal management device (14) and the access device (12), enabling the exchange of signals (SS) generated by the signal management device (14) and signals (SA) generated by the access device (12). In one embodiment, the signal management server (16) comprises the traffic channel (CT). In another embodiment, the creation of the traffic channel generates a direct connection between the devices.

The exchange of (SS) and (SA) signals assists more precisely the signal management device (14), since (SS) and (SA) signals are sent as signal sets for enabling greater stability of the flow of sent signals, according to the bandwidth capacity available on the access device (12).

In one embodiment, the process further comprises a step for processing in the signal management device (14) by reproducing the signals (SS) and (SA) in a overlapped way, where it is reproduced by a reproduction means (22), so as both signals (SS) and (SA) are perceived in the signal management device (14). In one embodiment, the signals (SA) are graphic coordinates and audio signals in order to aid the signal management device (14) indicating regions of interest.

In one embodiment, the signal management device (14) is configured to define operation modes. In a first mode, the signal management device (14) captures at least one signal (SE), the signal management device (14) further performs a step of compiling and later sending the signal (SU) for storage on the signal management server (16), where after storage, the server enables access of the compiled signals through at least one access device (12). In a second mode, the signal management device (14) sends a signal (SU) towards the signal management server (16) for authentication with at least one signal (SC) originating from at least one access device (12). If the authenticity of the connection is verified, the traffic channel (CT) enables communication between the signal management device (14) and the access device (12).

In one embodiment, the process of the invention is applied to professional procedures such as surgical procedures, clinical examinations, clinical analysis and/or medicine teaching. Training a surgery professional requires a lot of care, since an insecure professional may endanger the patient's life. Therefore, a specialist in the subject can provide a remote consulting using the teachings of the present invention, whereby the specialist being a consultant for the professional can instruct him in the tasks being performed.

Thus, the present invention shows a solution that enables the interconnection of an operator of a signal management device (14) with an user of an access device, so that the communication between the operator and the user occurs reliably, and wherein the user sends relevant signals (SA) to aid the operator which is where the action takes place.

### Examples

The examples herein shown are intended solely to exemplify one of the numerous ways of carrying out the invention, but without limiting the scope thereof.

### Example I

In a first example, as shown in Fig. 1, the signal management device (14) comprises two main intercommunicating components. The first component is a signal distributor (30) comprising capture means (31) for signal generation (SS), a processor as processing means (33), a command means (36) for signal generating (SU), a recording means (34), a signal transmission means (35), a reproduction means (22), a power source such that the signal distributor (30) generates signals (SS) and (SU) and decide the direction these signals are transmitted. The second component is a Front End (37) to assist in providing essential information to the surgical procedure performer.

Hence, the processor (33) is configured to perform processing of the signals (SS) and (SA) so as to overlap these signals in the reproducing means (22), where this processing comprises, for example, the application of mathematical tools to enable said overlapping signals.

Thus, the process implemented in this system is a surgery, such as laparoscopy or endoscopy. In another embodiment, the surgical process is any procedure performed by a healthcare professional in which an invasion/minimal invasion a patient's organism occurs.

### Example II

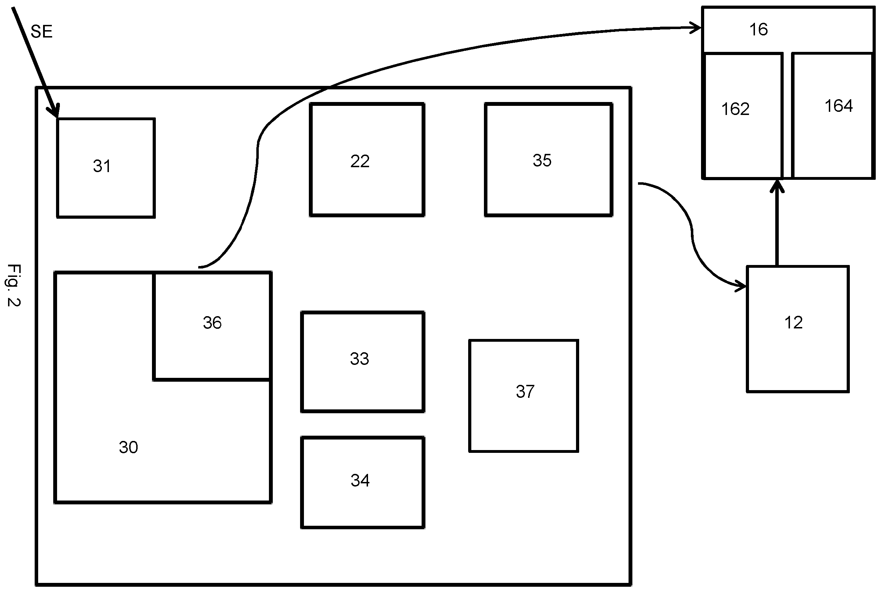

In another embodiment of the system, which is exemplified by Fig. 2, the signal management device (14) comprises a signal distributor (30), so that the received signals (SE) are properly distributed, and comprises a command means (36) which sends a signal (SU) for authentication of the connection between the signal management device (14) and the access device (12). In addition, the signal management device (14) comprises a video capture card (31) for receiving all signals (SE) from signal sources (10), a processor (33) for handling signals (SE) generating signals (SS), a recording means (34) for storing signals (SE), a signal transmission means (35) for transmitting signals (SS) and a power source.

In one embodiment, the processor (33) further comprises signal processing (SA) to enable overlapping of signals (SS) and (SA). In another embodiment, the signal management device (14) comprises another secondary processor for signal processing (SA) to enable overlapping signal (SS) and (SA). Furthermore, said processor may comprise multiple cores for execution the tasks in faster fashion.

The system further comprises an access device (12) and a signal management server (16) comprising a communication addressing means (162) and a storage means (164).

Also, the system of the present invention is able to interconnect the signal management device (14) with the access device (12) via a direct signal flow traffic channel (CT) without any other intermediate component in the system. For the creation of the traffic channel (CT), a communication authentication protocol is used, wherein the signal management device (14) uses the command means (36) to output at least one signal (SU) to request the connection, and the access device (12) outputs a signal (SC). In one embodiment, the protocol used is WebRTC.

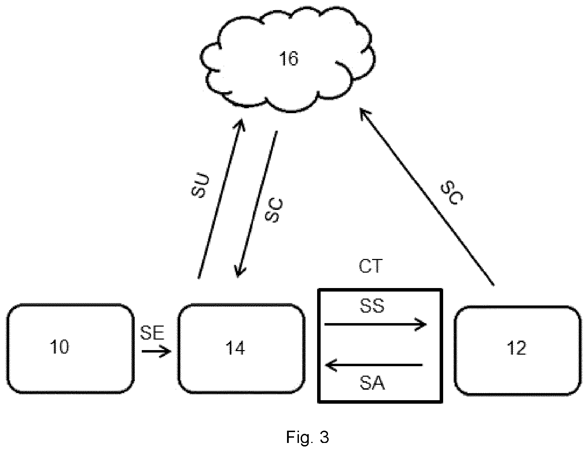

After authentication, the traffic channel (CT) is created without the need to continue a connection with the signal management server (16), thereby enabling the reduction of time from signal transmission to reception. In one embodiment, the traffic channel (CT) is comprised in the signal management server (16). In one embodiment, the traffic channel is a direct peer-to-peer, as shown in Fig. 3.

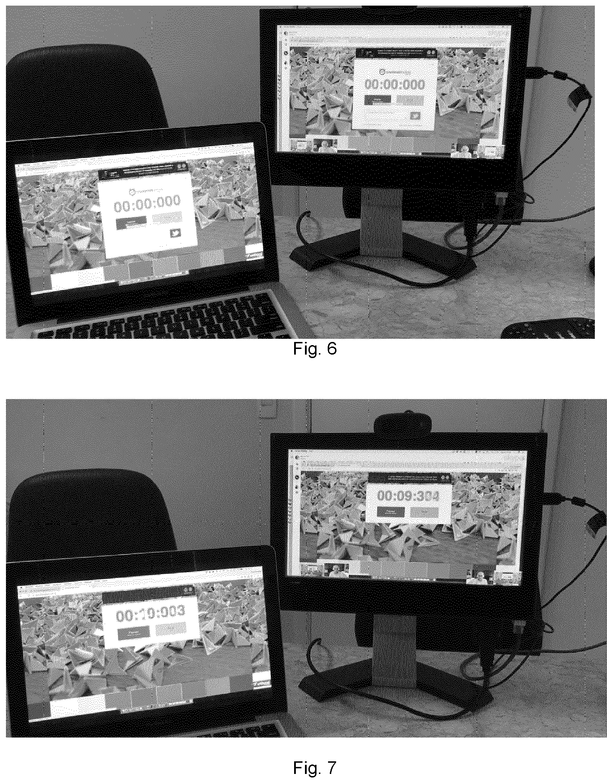

As can be seen in Figs. 6 and 7, a test was performed to measure the delay during communication between the devices, wherein a test was made with the management device (14) sending the signal (SS) to the access device (12), which returns the signal (SA), the time for this traffic to occur being calculated. To this purpose, it was found that this delay is of tenths to hundredths of a second, and it may further decrease depending on the processing capacity of the devices.

### Example III

In one embodiment, in addition to the storage function, the signal management server (16) comprises the selection function of the access device (12) provided to be able to instruct the signal management device (14), if there are one or more access devices (12) available for connection.

A signal management device (14) is understood as a signal provider (SS), and the access device (12) is understood to this signal receptor (SS), and the access device (12) is not only capable of viewing the signal (SS) and analyzing it, as well as returning a signal (SA) created by the access device (12) itself to the signal management device (14).

In one embodiment, the signal (SA) is understood as a data type returning to the signal management device (14) independently of the signal (SS), that is, it is not necessary that the signal management device (14) receives the signal (SS) again, and the access device (12) only sends the signal (SA). In one embodiment, the signal (SS) is a video to be sent by the signal management device (14), and in one embodiment, the signal (SA) is composed of audio and graphic coordinates that are returned to the signal management device (14) by the access device (12). In one embodiment, the signal (SS) is a full HD video. In one embodiment, the signal (SA) further comprises command signal for audio intensity control, that is, the access device (12) has a responsibility to regulate the volume of the transmission. In another embodiment, the signal (SC) directed to the signal management server (16) comprises audio intensity control command signal, that is, the access device (12) has the responsibility to regulate the transmission volume through command sent to the server (16). In one embodiment, the graphic coordinates created and routed via the signal (SA) to the signal management device (14) can be understood as an optimized telestration type.

### Example IV - Assisted Surgical Procedures

The signal management device (14) is used by a surgeon who is performing a surgical procedure and carrying a camera capable of capturing images of the surgical procedure. Thus, the surgeon is generating at least one video signal about the surgery performed, this video being routed via the signal (SS) to the remote access device (12). Thus, the consultant receives the video signal sent by the surgeon's device (14) and, in order to assist the surgeon, the consultant can send audio, images, videos signals, his own webcam video and perform graphic coordinates on the video signal (SS) which is received.

In the case of sending graphical coordinates, the consultant can graphically draw illustrations on the video signal (SS) he is receiving, in order to indicate/assist the surgeon with details of the procedure which is performed. Thus, the graphic coordinates drawn are captured by the access device (12) and coded so as it is possible to identify which position on the video display the illustrations should be made available. This enables the consultant to indicate to the surgeon the locations, for example, where an incision should be made during a particular surgical procedure.

For example, for coding the graphic coordinates a software/firmware arranged on the access device (12) is able to identify the pixels which have been changed from the consultant's illustrations and hence create a sequence of information (e.g. bits, qubits) indicating what are the pixel positions changed. Given this coding, the access device (12) generates the signal (SA) to send it to the signal management device (14) via the traffic channel (CT) created by the server (16).

Thus, the signal management device (14) receives this signal (SA) with the encoded data and thereby processes it so as the illustrations made by the consultant are correctly made available to the surgeon. For that, the signal management device (14) is provided with an algorithm implemented in processor (30) which identifies this information and reproduces the video signal with the pixels changed by the consultant, in order to faithfully reproduce the help illustrations generated by the consultant. Moreover, this process identifies the proportions of the video displays used, properly adjusting to the aspect ratios, performing, when necessary, upscaling and downscaling to adjust the video images so as the graphic coordinates reach the surgeon in the same way as they are drawn by the consultant.

The process described in this example can be used for any type of medical procedure using video signals, such as surgeries, examinations, clinical care etc.

### Example V

In one embodiment, the present invention further proposes to store a compilation of at least one signal (SE), generating a compiled file on a signal management server (16) so further viewing can be performed. In one embodiment, compilation is performed by capture means (31). In another embodiment, the compilation is performed by the processor (33). In another embodiment, the compilation is performed by recording means (34).

The signal management server (16) comprising the storage means (164) and the signal addressing means (162) receives the signals (SU) containing the compiled file. In one embodiment, this storage does not occur at the same time as the sending of the signals (SS) in order not to impair the quality and continuity of the communication signal, and therefore the storage occurs after the termination of the communication traffic channel (CT) of the signal management device (14) with the access device (12). In one embodiment, the file compiled together with the signal (SA) may also be stored on the signal management server (16). In one embodiment, the signal management server (16) comprises the signal selection function (SS) containing the contents of at least one signal (SE) available for output to the access device (12). In one embodiment, the signal management device (14) comprises the signal selection function (SS) containing the contents of at least one signal (SE) available for output to the access device (12).

The process described above is made possible by the capture means (31) generating signal (SS) used by the signal management device (14).

Fig. 2 shows an embodiment of the signal management device (14), wherein such a device is comprised of a signal distributor (30), capture means (31), recording means (34), reproducing means (22), command means (36), a power source, a processor (33) and a signal transmission means (35), wherein its components are interchangeable with each other.

The signal management device (14) is also responsible for capturing the signals (SA) and reproducing it in synchronization with the signal (SS). The signal source (10) is responsible for performing signal generation (SE). In one embodiment, the signal management device (14) comprises more than one signal source (10) as shown in Fig. 1, and there are therefore two possible signals (SE). In one embodiment, it is up to the access device (12) to select which signal (SS) it wants to watch and perhaps to send a signal (SA).

In one embodiment, two or more cameras may be used to provide multiple angles of an action. In one embodiment, the signal source (10) is a camera which will generate a video as a signal (SE). In one embodiment, wherein the signal (SS) is a video, in order to integrate with the current hospital system, the system in question comprises means of converting the acquired video into image frames for documentation of a surgical procedure following a standard.

Reproduction means (22) is responsible for receiving signal (SE) and signal (SA) and reproducing such information. In one embodiment, reproducing means (22) is a monitor and some audio spreading means.

The signal transmission means (35) is responsible for handling the signal (SS), sending it, receiving the signal (SA) in addition to send a compiled file for signal storage (SU) in a signal management server (16). Processor (33) interprets the signal (SA).

The capture means (31) in this example is a capture card (31) with the function of receiving the signal (SE) generated by the signal source (10) and sending to the processor (33) for generation a signal (SS).

The signal transmission means (35) corresponds to any means of input and/or output of signals traversing the traffic channel (CT). The signal transmission means (35) is associated with the processor (33).

In one embodiment, the processor (33) comprises a band analyzer, a decoder and connection to the signal distributor (30). The signal distributor (30) comprising decision-making means for signal routing. The processor (33) has the function of processing the signal (SS), storing it in the recording means (34), forwarding the signal (SS) coupled with the signal (SA) to the reproduction means (22) and forwarding signal processed for the signal transmission means (35). In one embodiment, the signal (SE) processed for transmission to the access device (12) is understood to be the signal (SS). In one embodiment, an IMX6 processor may be used.

The bandwidth analyzer has the function to analyze the bandwidth quality of the transmission channel bandwidth and to indicate to the processor in order to the processor adjust the signal to be sent. In one embodiment, the bandwidth analyzer is capable of indicating this resolution in order not to impair the transmission of the processed data to the access device (12). To this purpose, it may be needed the bandwidth analyzer to have to neglect and remove certain amounts of signal information (SS), which may be the least significant bits (or qubits) of information in order to make the signal to be sent something less complex, preventing interruptions and enabling greater reliability in communication between devices. In one embodiment, the bandwidth analyzer is able to relate the quality and availability of the network used to determine the information amount which must be sacrificed in order to maintain continuous communication.

From the indication made by the bandwidth analyzer, the present decoder receives the signal (SS), and encodes it reducing its complexity and making easy sending this information. As a coding product the signal (SE) becomes the signal (SS). In one embodiment, the codec used may be an H264.

Parallel to the process of generating the processed data, the processor (33) stores the signal (SE) in the recording means (34), which compiles the signal (SE) and makes the compiled file available to the command means (36), which in turn sends it to storage on the signal management server (16). In one embodiment, the signal (SE) compiled into a file is comprised as the signal (SU).

The signal (SS) is then routed to the transmission means, which is comprised as an output routing the signal (SS) to the access device (12). In one embodiment, the channel used for processed signal traffic is a network using any type of Peer-to-Peer protocol.

In another embodiment, upon receiving signal (SS), access device (12) generates signal (SA), wherein signal (SA) comprises inclusion of video and audio signals.

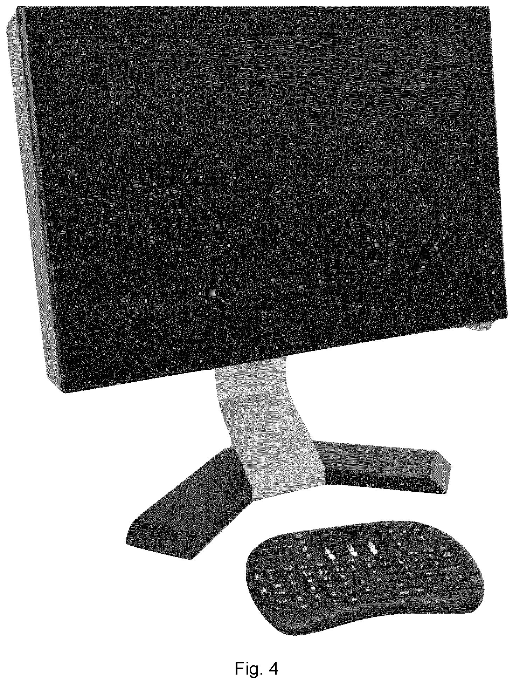

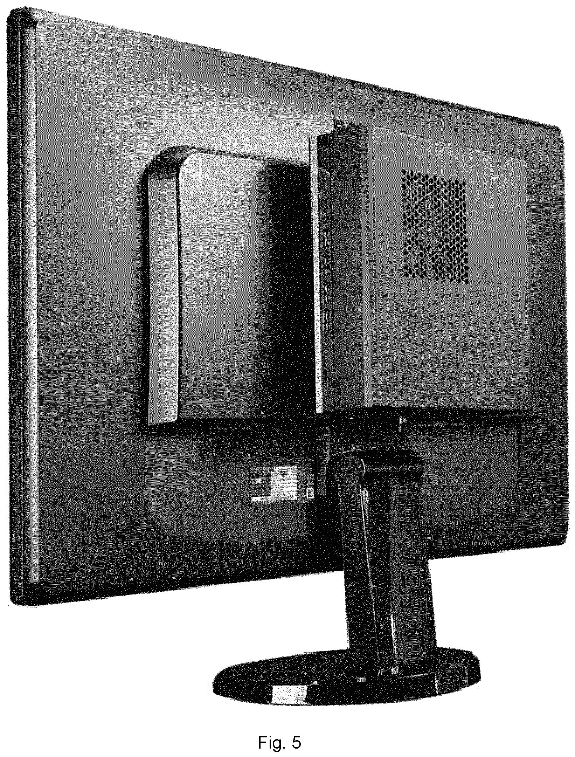

The signal (SA) is then routed to the signal management device (14) which in turn sends it to the processor (33), the processor having the function of receiving signal (SA) and synchronizing it with the signal (SS). Together the signal (SS) and signal (SA), the processor (33) thus routs this connection to the reproducing means (22) as shown in Figs. 4 and 5.

At the end of communication of the communicating device with the access device (12), and with the signal (SE) already stored in the recording means (34), the management device (14) sends the compiled file as a signal (SU) and routs the signal to the signal management server (16). In signal management server (16) the signal (SU) is stored in storage means (164). Thus, the server eventually becomes a kind of database, and the present invention determines this signal management server (16) is an accessible place for later retrieval of signals (SU) stored. In one embodiment, the stored files are the recorded full HD videos, and therefore they are stored on the signal management server (16), wherein they can be collected, downloaded for further analysis for educational purposes, for example. In one embodiment, the signals (SA) may also be stored at the signal management server (16) and may be synchronized with the corresponding signal (SU) stored.

In one embodiment, there may be more than one access device (12) connected to the signal management device (14). In this way, there may be a plurality of access devices participating at the same time. According to this embodiment, one of the access devices (12) would be a mediator organizing the participation of the other access devices present.

Those skilled in the art will appreciate the knowledge presented herein and may reproduce the invention in the embodiments disclosed and in other variants and alternatives within the scope of the following claims.

## Claims

1. System for performing and/or documenting of real-time remotely assisted professional procedure comprising:
a. at least one signal management device (14) comprising at least one between a capture means (31), a recording means (34), a reproducing means (22), a command means (36) and/or signal transmission means (35); and
b. at least one signal management server (16) comprising at least one between a storage means (164) and/or a communication addressing means (162);
- the signal management device (14) and the signal management server (16) communicate each other through a communication channel able to traffic at least one signal (SU);
- the system further comprising an access device (12) which communicates with the signal management server (16) having a user and/or consultant, wherein the access device (12) comprises means for sending of at least one signal (SC) for addressing request to the server;
**characterized by**
- the signal management server (16) generates a traffic channel (CT) between the access device (12) and the signal management device (14), said signal management device (14) sends a signal (SU) towards the signal management server (16) for authentication with at least one signal (SC) originating from at least one access device (12), wherein
the command means (36) outputs the signal (SU) to request the connection only after a command issued by a local user provided with the signal management device (14), said signal (SU) comprising at least a command signal for authentication by the signal management server (16); and
when the authenticity of the connection is verified, the traffic channel (CT) enables communication between the signal management device (14) and the access device (12).

2. System according to claim 1 **characterized in that** the capture means (31) of the signal management device (14) comprises means for receiving at least one signal (SE) from at least one signal source (10), wherein said capture means (31) generates at least one signal (SS).

3. System according to claim 1 **characterized in that** the access device (12) comprises a means for sending at least one signal (SA), said signal (SA) comprising at least one of:
- a video signal;
- an audio signal;
- graphic coordinates;
- overlapping images;
- a command signal for signal selection (SE);
- a command signal for audio intensity control;
- a text; or
- a combination thereof.

4. System according to claim 3 **characterized in that** the traffic channel (CT) comprises traffic from the SS and SA signals.

5. System according to claim 4 **characterized in that** the signal management device (14) comprises a processing means (33) configured to overlap and reproduce the SS and SA signals.

6. System according to claim 1 **characterized in that** the signal management device (14) compiles at least one signal (SE) thereby generating at least one file for later sending and storing at the signal management server (16), this file being accessed by the user and/or consultant through the access device (12).

7. System according to claim 1 **characterized in that** the system is applied for professional procedures including surgical procedures, clinical examinations, clinical analysis and/or teaching in the medical field.

8. Process for performing and/or documenting of real-time remotely assisted professional procedure comprising at least the following steps:
a. capturing of at least one signal (SE) from at least one signal source (10), by a capture means (31), in a signal management device (14);
**characterized by**
b. sending at least one signal (SU), said signal (SU) comprising at least a command signal for authentication, by the signal management device (14) to at least one signal management server (16) configured to receive the signal (SU) for authentication with at least one signal (SC) originating from at least one access device (12), where in the command signal for authentication is sent by the signal management device (14) only after a command issued by a local user provided with the signal management device (14);
c. generating of a traffic channel (CT) by the signal management server (16) after an authentication verification between the signal (SU) of the signal management device (14) and the signal (SC) of the access device (12), wherein the traffic channel (CT) generates communication between the signal management device (14) and the access device (12).

9. Process according to claim 8 **characterized in that** it further comprises a step of sending a signal (SC), by an access device (12), and further creating a traffic channel (CT), by the signal management server (16), connecting the access device (12) to the signal management device (14).

10. Process according to claim 9 **characterized in that** the traffic channel (CT) comprises signal (SS) generated by the signal management device (14) and signal (SA) generated by the access device (12).

11. Process according to claim 8 **characterized in that** it further comprises a processing step in the signal management device (14) overlapping reproducing the SS and SA signals.

12. Process according to claim 8 **characterized in that** it further comprises a step of compiling and later sending the signal (SU) for storage in the signal management server (16).

13. Process according to claim 8 **characterized in that** the process is applied in professional procedures such as surgical procedures, clinical examinations, clinical analysis and/or teaching in the medical field.

## Patentansprüche

1. System zur Durchführung und/oder Dokumentation eines ferngestützten professionellen Verfahrens in Echtzeit, umfassend:
a. mindestens eine Signalverwaltungsvorrichtung (14), umfassend mindestens eines zwischen einer Erfassungsvorrichtung (31), einem Aufzeichnungsmittel (34), einem Wiedergabemittel (22), einem Befehlsmittel (36) und/oder einem Signalübertragungsmittel (35); und
b. mindestens einen Signalverwaltungsserver (16), der mindestens eines von einem Speichermittel (164) und/oder einem Kommunikationsadressierungsmittel (162) umfasst;
- die Signalverwaltungsvorrichtung (14) und der Signalverwaltungsserver (16) kommunizieren miteinander über einen Kommunikationskanal, der in der Lage ist, mindestens ein Signal (SU) zu übertragen;
- das System umfasst ferner eine Zugriffsvorrichtung (12), die mit dem Signalverwaltungsserver (16) kommuniziert, der einen Benutzer und/oder Berater aufweist, wobei die Zugriffsvorrichtung (12) Mittel zum Senden von mindestens einem Signal (SC) zur Adressierungsanfrage an den Server umfasst;
**gekennzeichnet dadurch, dass**
der Signalverwaltungsserver (16) einen Verkehrskanal (CT) zwischen der Zugriffsvorrichtung (12) und der Signalverwaltungsvorrichtung (14) erzeugt, wobei die Signalverwaltungsvorrichtung (14) ein Signal (SU) an den Signalverwaltungsserver (16) zur Authentifizierung mit mindestens einem Signal (SC) sendet, das von mindestens einer Zugriffsvorrichtung (12) stammt, wobei
das Befehlsmittel (36) das Signal (SU) zur Anforderung der Verbindung nur nach einem von einem lokalen Benutzer, der mit der Signalverwaltungsvorrichtung (14) ausgestattet ist, ausgegebenen Befehl ausgibt, wobei das Signal (SU) mindestens ein Befehlssignal zur Authentifizierung durch den Signalverwaltungsserver (16) umfasst; und
wenn die Authentizität der Verbindung verifiziert ist, der Verkehrskanal (CT) die Kommunikation zwischen der Signalverwaltungseinrichtung (14) und der Zugriffsvorrichtung (12) ermöglicht.

2. System gemäß Anspruch 1, **dadurch gekennzeichnet, dass** die Erfassungsvorrichtung (31) der Signalverwaltungsvorrichtung (14) eine Vorrichtung zum Empfangen mindestens eines Signals (SE) von mindestens einer Signalquelle (10) umfasst, wobei die Erfassungsvorrichtung (31) mindestens ein Signal (SS) erzeugt.

3. System nach Anspruch 1, **gekennzeichnet durch** die Tatsache, dass die Zugriffsvorrichtung (12) ein Mittel zum Senden mindestens eines Signals (SA) umfasst, wobei das Signal (SA) mindestens eines der folgenden Elemente umfasst:
- ein Videosignal;
- ein Audiosignal;
- Grafikkoordinaten;
- überlappende Bilder;
- ein Befehlssignal zur Signalauswahl (SE);
- ein Befehlssignal zur Steuerung der Audiointensität;
- einen Text; oder
- eine Kombination daraus.

4. System gemäß Anspruch 3, **dadurch gekennzeichnet, dass** der Verkehrskanal (CT) Verkehr von den SS- und SA-Signalen umfasst.

5. System gemäß Anspruch 4, **dadurch gekennzeichnet, dass** die Signalverwaltungsvorrichtung (14) ein Verarbeitungsmittel (33) umfasst, das so konfiguriert ist, dass es die SS- und SA-Signale überlappt und reproduziert.

6. System nach Anspruch 1, **dadurch gekennzeichnet, dass** die Signalverwaltungsvorrichtung (14) mindestens ein Signal (SE) kompiliert und dadurch mindestens eine Datei zum späteren Senden und Speichern auf dem Signalverwaltungsserver (16) erzeugt, wobei der Benutzer und/oder Berater über die Zugangsvorrichtung (12) auf diese Datei zugreift.

7. System gemäß Anspruch 1, **dadurch gekennzeichnet, dass** das System für professionelle Verfahren, einschließlich chirurgischer Verfahren, klinischer Untersuchungen, klinischer Analysen und/oder Unterricht im medizinischen Bereich, angewendet wird.

8. Verfahren zur Durchführung und/oder Dokumentation eines professionellen Verfahrens mit Echtzeit-Fernunterstützung, das mindestens die folgenden Schritte umfasst:
a. Erfassen von mindestens einem Signal (SE) von mindestens einer Signalquelle (10) durch eine Erfassungsvorrichtung (31) in einer Signalverwaltungsvorrichtung (14);
**gekennzeichnet durch**
b. Senden von mindestens einem Signal (SU), wobei das Signal (SU) mindestens ein Befehlssignal zur Authentifizierung umfasst, durch die Signalverwaltungsvorrichtung (14) an mindestens einen Signalverwaltungsserver (16), der so konfiguriert ist, dass er das Signal (SU) zur Authentifizierung mit mindestens einem Signal (SC) empfängt, das von mindestens einer Zugangsvorrichtung (12) stammt, wobei das Befehlssignal zur Authentifizierung von der Signalverwaltungsvorrichtung (14) nur nach einem Befehl gesendet wird, der von einem lokalen Benutzer ausgegeben wird, der mit der Signalverwaltungsvorrichtung (14) ausgestattet ist;
c. Erzeugen eines Verkehrskanals (CT) durch den Signalverwaltungsserver (16) nach einer Authentifizierungsüberprüfung zwischen dem Signal (SU) der Signalverwaltungsvorrichtung (14) und dem Signal (SC) der Zugangsvorrichtung (12), wobei der Verkehrskanal (CT) eine Kommunikation zwischen der Signalverwaltungsvorrichtung (14) und der Zugangsvorrichtung (12) erzeugt.

9. Verfahren nach Anspruch 8, **gekennzeichnet durch** einen Schritt des Sendens eines Signals (SC) durch eine Zugriffsvorrichtung (12) und des weiteren Erzeugens eines Verkehrskanals (CT) durch den Signalverwaltungsserver (16), der die Zugriffsvorrichtung (12) mit der Signalverwaltungsvorrichtung (14) verbindet.

10. Verfahren nach Anspruch 9, **dadurch gekennzeichnet, dass** der Verkehrskanal (CT) ein Signal (SS), das von der Signalverwaltungsvorrichtung (14) erzeugt wird, und ein Signal (SA), das von der Zugriffsvorrichtung (12) erzeugt wird, umfasst.

11. Verfahren nach Anspruch 8, **dadurch gekennzeichnet, dass** es ferner einen Verarbeitungsschritt in der Signalverwaltungsvorrichtung (14) umfasst, der die SS- und SA-Signale überlappend reproduziert.

12. Verfahren nach Anspruch 8, **gekennzeichnet durch** einen Schritt des Kompilierens und späteren Sendens des Signals (SU) zur Speicherung im Signalverwaltungsserver (16).

13. Verfahren nach Anspruch 8, **gekennzeichnet durch** die Anwendung des Verfahrens in professionellen Verfahren wie chirurgischen Verfahren, klinischen Untersuchungen, klinischen Analysen und/oder Unterricht im medizinischen Bereich.

## Revendications

1. Système de réalisation et/ou de documentation d'actes professionnels assistés à distance en temps réel, comprenant :
a. au moins un dispositif de gestion de signal (14) comprenant au moins un moyen parmi un moyen de capture (31), un moyen d'enregistrement (34), un moyen de reproduction (22), un moyen de commande (36) et/ou un moyen de transmission de signal (35) ; et
b. au moins un serveur de gestion de signal (16) comprenant au moins un moyen parmi un moyen de stockage (164) et un moyen d'adressage de communication (162) ;
- le dispositif de gestion de signal (14) et le serveur de gestion de signal (16) communiquant entre eux via un canal de communication capable de faire transiter au moins un signal (SU) ;
- le système comprenant en outre un dispositif d'accès (12) qui communique avec le serveur de gestion de signal (16), ayant un utilisateur et/ou un consultant, dans lequel le dispositif d'accès (12) comprend un moyen pour envoyer au moins un signal (SC) afin d'adresser une demande au serveur ;
**caractérisé en ce que**
- le serveur de gestion de signal (16) génère un canal de trafic (CT) entre le dispositif d'accès (12) et le dispositif de gestion de signal (14), ledit dispositif de gestion de signal (14) envoie un signal (SU) au serveur de gestion de signal (16) pour l'authentification, avec au moins un signal (SC) provenant d'au moins un dispositif d'accès (12), dans lequel
le moyen de commande (36) émet le signal (SU) pour demander la connexion uniquement après une commande émise par un utilisateur local muni du dispositif de gestion de signal (14), ledit signal (SU) comprenant au moins un signal de commande pour une authentification par le serveur de gestion de signal (16) ; et
quand l'authenticité de la connexion est vérifiée, le canal de trafic (CT) permet une communication entre le dispositif de gestion de signal (14) et le dispositif d'accès (12).

2. Système selon la revendication 1, **caractérisé en ce que** le moyen de capture (31) du dispositif de gestion de signal (14) comprend un moyen pour la réception d'au moins un signal (SE) provenant d'au moins une source de signal (10), dans lequel ledit moyen de capture (31) génère au moins un signal (SS).

3. Système selon la revendication 1, **caractérisé en ce que** le dispositif d'accès (12) comprend un moyen d'envoi d'au moins un signal (SA), ledit signal (SA) comprenant au moins l'un des éléments suivants :
- un signal vidéo ;
- un signal audio ;
- des coordonnées graphiques ;
- des images superposées ;
- un signal de commande pour une sélection de signal (SE) ;
- un signal de commande pour un contrôle d'intensité audio ;
- un texte ; ou
- une combinaison de ceux-ci.

4. Système selon la revendication 3, **caractérisé en ce que** le canal de trafic (CT) comprend un trafic provenant des signaux SS et SA.

5. Système selon la revendication 4, **caractérisé en ce que** le dispositif de gestion de signal (14) comprend un moyen de traitement (33) configuré pour superposer et reproduire les signaux SS et SA.

6. Système selon la revendication 1, **caractérisé en ce que** le dispositif de gestion de signal (14) compile au moins un signal (SE), générant ainsi au moins un fichier destiné à être envoyé et stocké ultérieurement sur le serveur de gestion de signal (16), l'utilisateur et/ou le consultant ayant accès à ce fichier via le dispositif d'accès (12).

7. Système selon la revendication 1, **caractérisé en ce que** le système est appliqué à des actes professionnels incluant des interventions chirurgicales, des examens cliniques, des analyses cliniques et/ou des enseignements dans le domaine médical.

8. Processus de réalisation et/ou de documentation d'un acte professionnel assisté à distance en temps réel, comprenant au moins les étapes suivantes :
a. capture d'au moins un signal (SE) provenant d'au moins une source de signal (10) par un moyen de capture (31), dans un dispositif de gestion de signal (14) ;
**caractérisé par**
b. l'envoi d'au moins un signal (SU), ledit signal (SU) comprenant au moins un signal de commande pour l'authentification, par le dispositif de gestion de signal (14), à au moins un serveur de gestion de signal (16) configuré pour recevoir le signal (SU), pour une authentification avec au moins un signal (SC) provenant d'au moins un dispositif d'accès (12), dans lequel le signal de commande pour l'authentification est envoyé par le dispositif de gestion de signal (14) uniquement après l'envoi d'une commande par un utilisateur local équipé du dispositif de gestion de signal (14) ;
c. la génération d'un canal de trafic (CT) par le serveur de gestion de signal (16) après une vérification d'authentification entre le signal (SU) du dispositif de gestion de signal (14) et le signal (SC) du dispositif d'accès (12), dans lequel le canal de trafic (CT) génère une communication entre le dispositif de gestion de signal (14) et le dispositif d'accès (12).

9. Processus selon la revendication 8, **caractérisé en ce qu'**il comprend en outre une étape d'envoi d'un signal (SC) par un dispositif d'accès (12), et de création en outre d'un canal de trafic (CT) par le serveur de gestion de signal (16), qui connecte le dispositif d'accès (12) au dispositif de gestion de signal (14).

10. Processus selon la revendication 9, **caractérisé en ce que** le canal de trafic (CT) comprend un signal (SS) généré par le dispositif de gestion de signal (14) et un signal (SA) généré par le dispositif d'accès (12).

11. Processus selon la revendication 8, **caractérisé en ce qu'**il comprend en outre une étape de traitement dans le dispositif de gestion de signal (14) qui superpose la reproduction des signaux SS et SA.

12. Processus selon la revendication 8, **caractérisé en ce qu'**il comprend en outre une étape de compilation et ensuite d'envoi du signal (SU) pour son stockage sur le serveur de gestion de signal (16).

13. Processus selon la revendication 8, **caractérisé en ce que** le processus est appliqué dans des actes professionnels tels que des interventions chirurgicales, des examens cliniques, des analyses cliniques et/ou des enseignements dans le domaine médical.
